# EUROPEAN PATENT SPECIFICATION

(11) **EP 1 626 778 B1**
(45) Date of publication and mention of the grant of the patent: **17.03.2010**
(21) Application number: 04731110.5
(22) Date of filing: 04.05.2004
(51) Int. Cl.: A61N 5/067

(54) **LASER APPARATUS FOR HEAT TREATMENT**
LASERGERÄT FÜR DIE WÄRMEBEHANDLUNG
APPAREIL LASER POUR TRAITEMENT THERMIQUE

(30) Priority: 05.05.2003 EP 03010117; 06.05.2003 US 320167 P
(43) Date of publication of application: 22.02.2006
(73) Proprietor: Trion-Swiss Limited, 3174 Thörishaus (CH)
(72) Inventor: LAGERGREN, Håkan, 100 28 Stockholm (SE); LUNDEBERG, Thomas, 181 31 Lidingö (SE)
(74) Representative: Herbjörnsen, Rut
(86) International application number: PCT/SE2004/000677
(87) International publication number: WO 2004/098710

(56) References cited:
- WO-A-03/022189
- FR-A1- 2 827 151
- RU-C1- 2 214 844
- US-A- 4 633 875
- US-A- 5 021 452
- US-A1- 2003 002 297
- US-B2- 6 503 269

## Description

### Technical Field

The present invention relates in general to apparatus for heat treatment of skin, in particular without evoking pain. More particularly, the present invention relates to an apparatus for non-contact stimulation by heat of receptors in the skin by means of laser radiation of skin areas on a patient for achieving biological effects.

### Background

Pain and pain relief is an important issue in health care. Traditionally, analgesic drugs have been commonly used for pain relief. However, there are drawbacks with the use of drugs. It is for example usually important to treat and eliminate the cause of pain, and analgesic drugs may contribute to conceal the actual cause and hamper efficient diagnosing. In other cases, the cause of the pain is known as a part of a normal healing process for example after a surgical operation. Another more and more frequently occurring situation is the presence of more or less chronic pain due to wear and tear in e.g. aged people, due to a systemic disease process or for unknown reasons. Many of the patients that have chronic pain show little or no response to analgesic pain treatment, and there is also a risk for addiction or intoxication in long term use of analgesic chemicals.

Alternative methods have therefore emerged and laser radiation for the treatment of acute and chronic pain is now a fairly well established procedure. Laser radiation generates heat in the radiated tissue and the heat in its turn causes physiological pain relieving effects.

The physiological background and the mechanisms for response to heat are as follows. Primary nociceptive afferents, i.e. nerve paths that lead signals from pain receptors in tissue to the central nervous system, provide an input to the central nervous system that may lead to pain sensations and nociceptive reflexes through efferents, i.e. nerve paths leading from the central nervous system. An example of such a nociceptive reflex is muscle contraction to remove a limb from a source of pain. The afferents are also involved in the local control of vascular functions, such as vascular dilation in a limited tissue area. This dual afferent and efferent role of primary nociceptive afferents is related to the release of neuropeptides at the central terminals as well as at the peripheral terminals of these so called peptidergic sensory neurons. This is known as one of the origins of Dale's priciple, which says that an identical chemical transmitter is liberated at all the functional terminals of a single neuron.

The peripheral release of substance P, which is a vasoactive intestinal peptide that inter alia induces vasodilation and increases capillary permeability, and calcitonin gene related peptide (CGRP) causes hyperaemia, i.e. an excess of blood, by dilation of arterioles and plasma extravagation by means of leakage at postcapillary venules. These peripheral consequences of nociceptor activation are collectively termed neurogenic inflammation, since they present the cardinal signs of inflammation, i.e. calor (heat), rubor (redness), tumor (swelling) and dolor (pain).

There is normally a vasoconstriction due to sympathetic innervation and circulating catecholamines. The vasodilatation component of neurogenic inflammation is in addition to endothelium dependent mechanisms and metabolic vasodilation one of several mechanisms that counteract the vasoconstriction. Recent studies using electrical stimulation of the sympathetic chain and the dorsal roots suggest that nociceptor mediated vasodilation may even be the most powerful vasodilator mechanism in the skin.

The most typical primary nociceptive afferent that has been shown to be involved in both neurogenic inflammation and pain sensation is the C fibre polymodal nociceptor, which responds to noxious mechanical, chemical, cold and heat stimuli. Heat responses of these nociceptors have been well characterised in previous studies, and therefore heat stimuli are most suitable for analysis of the relationship of nociceptor discharges, neurogenic inflammation and pain sensation. Nevertheless, most studies so far have used chemical or electrical stimuli to elicit neurogenic inflammation rather than using noxious heat. In studies using heat stimulation, the reported threshold temperatures for the vasodilation component of neurogenic infalmmation differ widely in the range between 36,5 °C to 55 °C. Also the maximum skin temperatures that did not elicit any vasodilation range from 35 °C to 40,5 °C.

To summarise, the effect and mechanism of heat treatment by means of laser radiation is fairly well known, but there is a need for an improved control of stimulation and heat parameters in order to achieve biological effects, such as pain relief.

### Prior Art

Examples of prior art are disclosed in the following patent documents.

US 5,021,452 shows a process for improving wound healing, comprising administering ascorbate to a wound site and irradiating the wound site with a low-power laser at wave length of about 600 nm to about 1100 nm.

US 2003/0002297 shows an apparatus provided with laser diodes mounted in a watch-case and devised for irradiating a skin area for the purpose of achieving a therapeutic effect.

Document FR-A-2 827 151 relates to a method of controlling the tissue temperature induced by a surgical laser. The method comprises: a step involving the sampling of at least two wavelengths which originate from the tissue during treatment and which are longer than the wavelength emitted by the laser; and a step in which a ratio is calculated between the two sampled wavelengths, the value of the tissue temperature being correlated with the values of the ratio.

The document discloses also an apparatus for carrying out the method.

### Problem to be Solved by the Invention

The general problem that the invention seeks to solve is to provide an improved apparatus for efficiently achieving biological effects by means of heat treatment, and in particular pain relief.

Aspects of the problem is:
To determine parameters for controlling a heat treatment apparatus in order to achieve an efficient treatment.
To provide safety control in the heat treatment.
To provide a heat treatment apparatus with a laser source for heat generation and appropriate control thereof.

### Object of the Invention

It is therefore a general object of the present invention to provide an improved apparatus devised for treatment of skin with heat, in particular without evoking pain.

### Summary of the Invention

The object of the invention is achieved and the problem is solved by means of an apparatus for non-contact stimulation by heat of various receptors in the skin in order to achieve a biological effect, for example pain relief in a living being. The invention is based on heat generation in or heat exposure of mechanoreceptors, warmth receptors and heat receptors in a surface structure, for example the skin of a patient. Different exposure schemes are provided in the apparatus to be used in therapeutic treatment of different parts of the skin on a patient. Preferably, heat exposure or heat generating energy is directed toward skin areas of trigger points or acupuncture points.

The temperature in a heat treated skin area is monitored by means of a temperature sensor detecting the current temperature of the skin. Preferably, the skin temperature is detected before, during and after laser irradiation. The skin temperature is input to a feedback control loop that is coupled to control the laser irradiation such that emitted laser light achieves a skin temperature within predetermined values or thresholds in order to continuously adapt the irradiation. The inventors have found that a healing process of the organism is triggered and/or stimulated by exposing various receptors in the skin to heat in the range of 40-42 °C for a short period of time. Different other advantageous temperature ranges are described in conjunction with embodiments of the invention below.

In one variety of the invention, heat is generated in the tissue comprising the receptors by means of pulsed, defocussed laser radiation. Different radiation schemes are provided in the apparatus to be used in therapeutic treatment of different parts of tissue, for example the skin on a patient. However, care must be taken to control the heat exposure or energy radiation such that injuries or damage to the tissue are avoided.

The inventive concept is below explained by means of an exemplifying embodiment with a laser source for heat generation in tissue.

It should be emphasised that the term "comprises/comprising" when used in this specification is taken to specify the presence of stated features, integers, steps or components but does not preclude the presence or addition of one or more other features, integers, steps, components or groups thereof.

### Brief Description of the Drawings

The invention will be further explained with reference to the accompanying drawings, in which:
Fig 1 shows a schematic overview of an embodiment of the invention with a laser source and a control apparatus;
Fig 2 and Fig 3 show schematic diagrams of physiological relations upon which the invention is based;
Fig 4 shows an exemplifying diagram of temperature sweeping in one mode of operation of the invention; and
Fig 5 shows a schematic drawing of an exemplifying realisation of the invention in the shape of a laser treatment apparatus.

### Detailed Description of Embodiments

### Heat stimuli vasodilation

The inventors of the apparatus of the present invention have found that mild heat stimuli cause vasodilation that may spread for at least 30 millimetres from the heated skin site. It is currently believed that this dilation is mediated by the release of vasoactive peptides, such as substance P and CGRP from a sub-population of C fibre nociceptors via an axon reflex mechanism. When skin temperature is raised slowly, thus avoiding high phasic discharge rates, the threshold for pain perception may be several degrees above that for neurogenic vasodilation. This dissociation of pain and vasodilation thresholds is related to summation at central synapses, and is used for a non-painful test of nociceptor function.

The inventors have found that about 50 % of the presumed maximum increase in blood flow due to local heating occurred in surrounding unheated skin, and therefore suggest that release of vasoactive neuropeptides from C fibre nociceptors also plays a major role in the prefusion of the heated tissue itself.

The parameters of the minimum heat stimuli that are capable of eliciting neurogenic vasodilation in human hairy skin have been assessed in experimental studies.

### Heat stimulation

The apparatus in accordance with an embodiment of the inventive concept comprise applying heat stimulation by means of laser radiation. The laser is devised such that is emits a laser light having a wavelength that is known not to cause any skin trauma, such as burning. A currently preferred laser is a CO₂ laser that operates at a wavelength of 10600 nanometers (nm). Other examples of laser that are useful for the purpose of the invention are GaAs laser, Nd-YAG laser or Dye-laser. Important is however to adapt control parameters such as the output power, scan rate and the like in accordance with the invention.

The apparatus of the invention may be used an examination phase and a therapy phase. In both phases, laser radiation is applied to the skin of the patient and the skin temperature is monitored. This is preferably done by infrared detection of heat that is emitted by the skin surface. However, other methods and devices for monitoring the skin temperature are also conceivable within the inventive concept. For safety reasons and control purposes the current skin or surface temperature is compared to thresholds adapted to different control parameters. For patient safety, the current skin or surface temperature is compared to a predetermined threshold for a maximum allowed skin or surface temperature. If the maximum skin or surface temperature threshold is attained or exceeded, the radiation is stopped or the radiation intensity is reduced.

### Examination and therapy

In the examination phase, the patient is examined and the patient's current pain thresholds are determined. The heat generating exposure, for example laser radiation is controlled such that the skin temperature is swept preferably in the range of about 35°C to about 50°C.

In the therapy phase, the heat generating exposure, for example laser radiation is controlled such that the skin temperature is swept preferably in the range of about 35 °C to about 45°C.

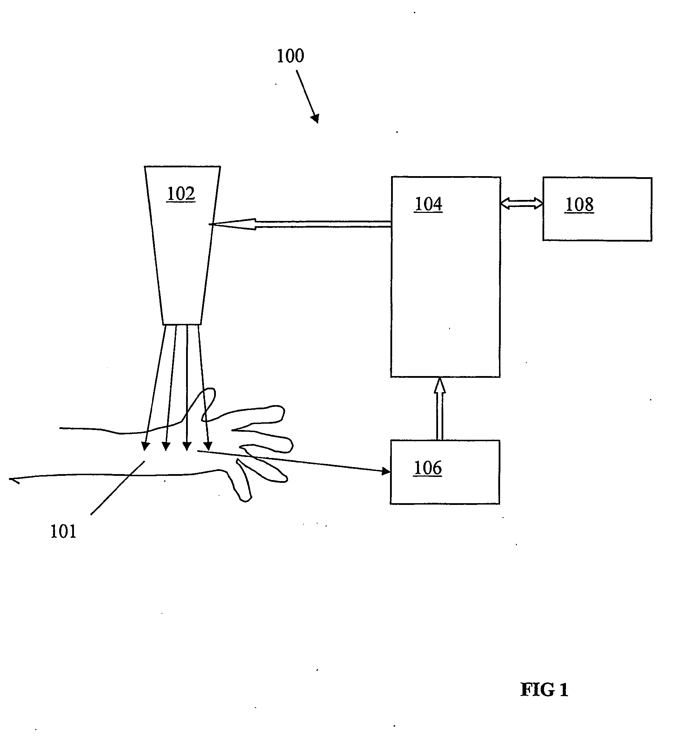

The heat generating exposure, for example laser radiation is carried out such that a contact free heat stimulation of skin receptors is achieved while the obtained skin temperature is monitored. **Fig 1** shows an embodiment of a laser apparatus 100 in accordance with the invention. A laser light source 102 emits laser light to a skin area 101 of a patient and stimulates the receptors of the skin by scanning or sweeping the light beam over the skin area. The laser light source is coupled to a control unit 104 that is adapted to control the laser light source with respect to a number of parameters such as emitted light intensity, pulse frequency, power of emitted energy, scanning etc. The apparatus comprises a temperature sensor devised to detect the temperature of the skin area that is radiated with laser light. In one embodiment the temperature sensor is realised by means of an infrared sensor that is configured to detect heat emitted from the radiated skin area. Other sensors are also conceivable, however they have capable of being mounted or adjusted such that the temperature of the skin area that receives the laser radiation is detected. In one embodiment wherein the laser beam for example is scanned over a skin area, the apparatus is devised such that a first temperature value of the treated skin area is detected before the laser beam hits the skin, a second temperature value is detected during irradiation i.e. in the moment the laser beam hits the skin and a third temperature value is detected after the irradiation has ceased. These temperature values are used in a feedback control loop devised to adapt the radiation to achieve a predetermined temperature or temperature range in the treated skin area.

An output of the skin temperature sensor 106 is coupled to the control unit 104. The control unit 104 is in its turn provided with a comparator (not shown) configured to compare the skin temperature signal with predetermined or selectable thresholds as explained above. The output of the comparator is input to a feedback control loop configured to control the output of the laser, such that the skin temperature is kept within a selected temperature range or below a maximum allowed temperature. The control unit 104 is further coupled to an input/output interface 108 configured for enabling presentation, operation and control of parameters of the laser apparatus 100. The laser light source 102 is coupled to or comprises a scanner mechanism configured to scan the laser beam across a selected area of skin to be stimulated or treated.

The heat stimulation is preferably carried out by subjecting the skin tissue to a pulsed laser light, where the pulse rate of the laser light is controlled to be in the range of about 0,5 to 400 Hertz (Hz). The frequency range is varied dependent on the characteristic of the skin tissue that is treated, and in one embodiment the invention employs a more narrow range in the stimulation pulse rate of about 80 to 200 Hz or even in the range of 200 Hz. The wavelength of the laser light should preferably be more than about 340 nm, and preferably about 10600 nm.

An embodiment of the laser control is adapted to pulse the laser light with a pulse rate in the range of about 1000 Hz to about 10 000 Hz. The stimulation of skin receptors is achieved by intermittently interrupting or chopping the laser light for periods in range of about 1-5 milliseconds (ms), preferably about 3 milliseconds, such that the rate of interruptions has a frequency in the range of about 0,5 to 400 Hz. In different embodiments, the rate of interruptions is adapted to the range of about 80 to 200 Hz or to the preferred range of 200 Hz. In this manner the stimulating light is applied as burst of light pulses between interruptions of radiating light having said frequencies. This manner of varying the rate of interruptions is useful as an alternative manner of controlling the laser light output.

Different skin receptors are stimulated at different temperatures and the laser apparatus is controlled to achieve that effect. There are three currently known receptors in human skin, namely 1. Mechano-receptors, 2. thermo receptors (also called warm receptors) and 3. pain receptors. The pain receptors are sensitive to heat, and therefore they are also called heat receptors in this text. Some areas of the skin are populated by mixed receptors, i.e. different combinations of the previous three kinds of receptors with receptors 1 +2, 1+3, 2+3 or 1+2+3. with the apparatus of the invention, the different receptors or receptor combinations are radiated with different radiation characteristics and the laser apparatus is controlled accordingly.

Thermal receptors or warm receptors in primates have been conclusively established to have the following characteristics:
Maintained discharge at constant skin temperatures, with a discharge rate that is proportional to skin temperature (also called static response).
Insensitive to non-thermal stimuli.
Small receptive fields in the skin of about 1-2 millimetres or less in extension. Each afferent fibre supplies only one or a few warm sensitive points.
As an example, in human skin tissue there are about 0,4 warm sensitive points per square centimetre in the hands whereas there are more than 20 warm sensitive points per square centimetre in the facial skin tissue.

Heat receptors convey a heat sensation that is often painful. The heat sensation that occurs regularly at skin temperatures at or above 45 °C is not yet entirely understood in terms of neurophysiological correlates. It appears however, that there are specific heat receptors distinguished from the thermo receptors. The painful character of heat sensation, similar to that of acupuncture, and the fact that heat stimuli are injurious, suggest that heat sensation is more appropriately considered a quality of pain rather than a quality of thermo reception. Autonomic reflexes are triggered by heat stimulation, for example oppressive closeness results in sweating and vasodilation whereas chill results in shivering and vasoconstriction. The effect obtained by stimulating heat receptors is the same as those obtained with acupuncture or moxibustion. The number of pain sensitive points in skin tissue is more than 1000 points per square centimetre, and thus local treatment of skin areas in the magnitude of a few square centimetres can be applied. An embodiment of the invention for this purpose is configured to apply a laser light stimulation at a low frequency in the range of about 1 to 2 Hz to maintain a constant temperature of about 45 °C, or even in the range between about 45 to 50 °C. A maximum allowable skin temperature is in this case suitable to set to about 50 °C. It is also conceivable to sweep the laser light at for example 2 Hz, 15 Hz or 24 Hz.

Mechano receptors appear in hairy as well as hairless skin of man in three types, namely pressure receptors, touch receptors and vibration receptors, dependent on the characteristics of the stimulus that these receptors respond to. The thermo receptors and the heat receptors can also be classified in an analogous manner and below the different classifications are discussed for all three kinds of receptors.
*Pressure and heat receptors* are responsive to the intensity of stimulation and thus have the function of intensity detectors. The discharge rate of these receptors is proportional to the stimulus intensity at each point in time during the stimulus. In a double logarithmic co-ordinate system, the relation between stimulus intensity I and discharge rate can be represented by a straight line at all times, which indicates that this relation is a power function of the form R=I^{a}. *Touch and heat receptors* are responsive to the velocity of a heat propagation of heat stimulus (due to pulse frequency) or an indentation of the skin, i.e. skin displacement, where the receptor is located. In a double logarithmic co-ordinate system, the impulse rate is a linear function of indentation velocity (i.e. the first derivative of distance with respect to time). The relation between discharge rate of the receptor and indentation velocity is thus also described by a power function similar to that mentioned above, but during square-wave stimuli. These receptors adapt and decline in the frequency of firing of a neuron within 50-500 milliseconds, under conditions of constant stimulation.
*Vibration receptors* can be excited by mechanical or heat/wann stimuli and are responsive to the acceleration of skin displacement in indentation stimulation, or of the acceleration of heat propagation due to the pulse frequency in heat and warm stimulation. Stimuli of a magnitude equal to the threshold intensity, and thus several times more powerful than stimuli that is required for a response in the previous mechano-receptors, elicit one impulse in vibration receptors and they adapt very rapidly. Therefore, vibration receptors can neither signal the depth nor signal the velocity of indentation or heat stimulus. However, with sinusoidal stimulation each cycle of the sine wave elicits an action potential. The minimum amplitude of the sinusoidal oscillation of the stimulation that is necessary to give a 1:1 response decrease greatly as the stimulus frequency rises. Thus, in a double logarithmic plot the slope of the line that depicts a relation between the threshold amplitude S to stimulus frequency f has been shown to be about minus two in the frequency range of 30 to 200 Hz. This relation can be written as S=constant *f. This indicates that the adequate stimulus to these receptors is the second derivative of indentation depth with respect to time, i.e. the acceleration of skin displacement or heat propagation due to the pulse frequency of heat and warm stimulation. The latter has not been shown in detail, but experimental trials indicate this mechanism. At frequencies above about 200 Hz, the threshold for exciting the vibration receptors rice again, and at frequencies above about 400 Hz it is no longer possible to elicit impulses in a 1:1 relation.

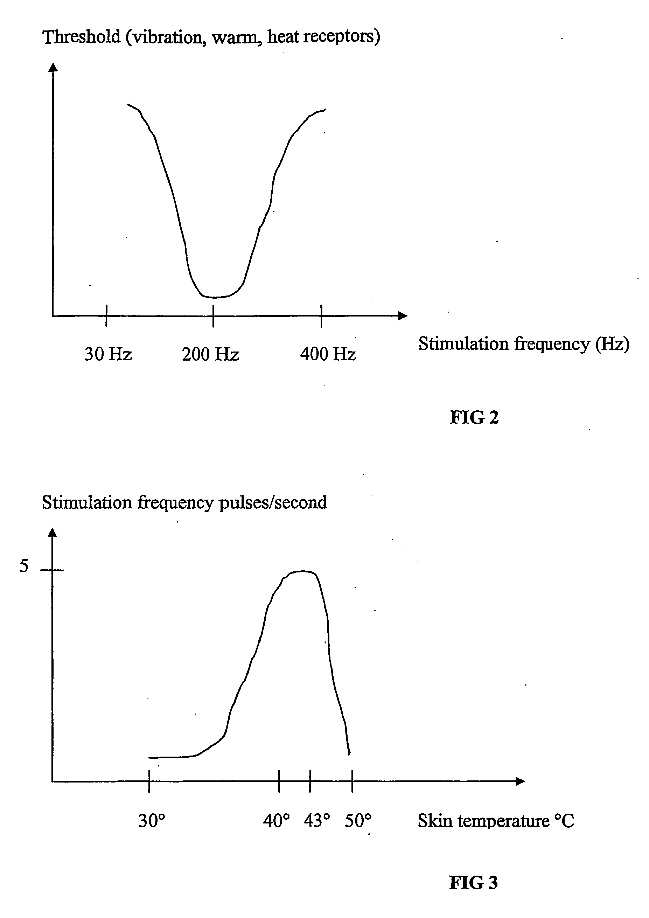

**Fig 2** shows schematically in a diagram the relation between stimulation frequency and the stimulation threshold for vibration, warm and heat receptors. As shown, the threshold has a minimum for a stimulation frequency in the range of 200 Hz, and therefore embodiments of the invention is preferably operated at this frequency.

**Fig 3** shows schematically in a diagram the relation between pulse frequency in pulses per second of the stimulation light and the attained skin temperature in °C. There is a maximum in the pulse frequency of about 5 pulses/second, which generates a skin temperature in the range of 40 to 43 °C.

The radiation of laser light in order to provide a heat stimulus is controlled dependent on the dominating kind of receptors in the treated skin area in accordance with the above described response pattern. Thus:
Mechano-receptors are preferably radiated with a laser light intensity in the range of about 1 to 5 Watt (W) at a rate of about 200 Hz in order to keep the skin temperature in the range of about 40-42 °C.
Warm receptors are preferably radiated with a laser light intensity in the range of about 5 to 10 Watt (W) at a rate of about 5 Hz in order to keep the skin temperature in the range of about 42-43°C.
Heat receptors are preferably radiated with a laser light intensity in the range of about 10 to 15 Watt (W) at a rate of about 200 Hz in order to keep the skin temperature in the range of about 45 °C.
In rare cases, a stimulation configured to keep the skin temperature at about 45 °C, preferably with a stimulation frequency of 200 Hz, is beneficial.

The power of the stimulus intensity as expressed in Watt in the previous passage is not a suitable control parameter since the required power depends on the size of the area that is radiated, the duration of the stimulation and the distance of radiation. The latter distance thus being the distance from the laser source to the radiated area. Therefore, the radiation of the laser apparatus is controlled dependent on the stimulation frequency, the stimulation area and the distance of radiation and attained skin temperature. Preferably, the apparatus is controlled to maintain a substantially constant skin temperature independent of frequency and the size of the skin area that is exposed to radiation.

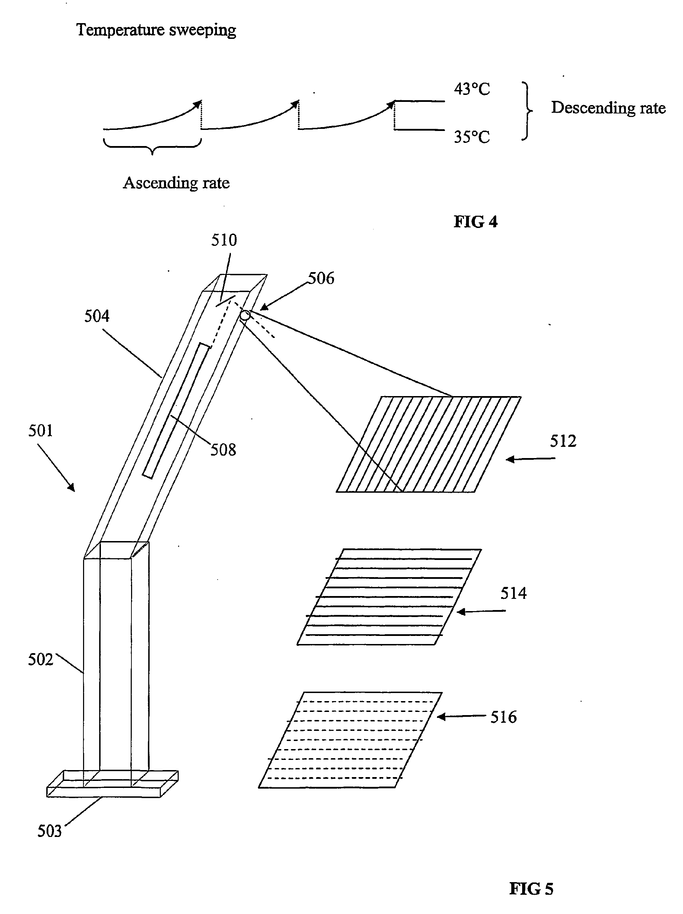

The invention further takes account of the spatial resolution of pressure, touch and vibration sensations in skin. The stimulation thresholds, that is the stimulation at which a sensation is perceived, are generally increasing due to the adaptation mechanism of receptors. The stimulation thresholds are distinctly lower in receptors that are stimulated successively over a spatial skin area, than the stimulation thresholds in receptors that are simultaneously stimulated. In another wording, sequential stimuli are better resolved than simultaneous stimuli. The thresholds in successive stimulation are often only about a quarter of the thresholds in simultaneous stimulation. So, for example, in indentation stimulus the successive stimulation thresholds are typically about 1 millimeters compared to 4 millimeters of simultaneous stimulation thresholds. This phenomenon is also reflected in the fact that surface characteristics of an object can be determined considerably more easily by stroking it than by touching it without movement. In order to utilise this in the invention, the stimulating laser light is swept (scanned) and preferably pulsed with a low frequency in the range of about 1 to 2 Hz. When sweeping or scanning the laser light over a skin area, the laser light is for example oscillated around a centre point with an amplitude of e.g. 10-20 centimetres in a first direction and e.g. 10-20 centimetres in a second direction. In this example, the laser stimulus is thus approximately sweeping over a substantially rectangular area of about 100 - 400 square centimetres. The successive stimulation can also be achieved by sweeping the temperature. **Fig 4** illustrates a sweeping stimulation pattern devised such that the attained skin temperature is swept between 35 and 43 °C. In this example, the stimulation is controlled to ascend with 1 °C per second and to descend with 8 °C per second.

In therapy the area stimulated area is adapted to what is suitable for the body part. For example, when treating a skin area in the face a comparatively small area is swept with laser light and the apparatus is applied at a short distance from the patient. When treating skin areas on other body parts a comparatively large area is radiated with laser light. Stimulation is advantageously directed towards trigger points or acupuncture points.

A therapy session would comprise the steps of:
1. Diagnosing the patient.
2. Placing the patient on a bench or chair.
3. Applying the light emitting part of the apparatus to the diagnosed body part in front of a selected skin area.
4. Selecting a suitable predetermined control program for selected mode of operation of the apparatus.
5. Starting stimulation output of apparatus dependent on selected control program.
6. Sweeping the laser beam of the apparatus over the skin area.
7. Increasing the power of the laser light until the patient signals that the induced heat is perceived as maximum persistable.
8. Continuing treatment for a period of time in the range of about 1 to 60 minutes.
The number of treatment sessions are varied dependent on the diagnosis and the healing process of the current injury. The diagnosis is evaluated by means of a pain evaluation method or by means of the patient's own perception of pain level.

**Fig 5** shows schematically an embodiment of a laser treatment apparatus 501 in accordance with the invention and which would comprise the components shown in fig 1. A laser support arm 504 is mounted on a support stand 502 comprising a base plate 503 that may be provided with not shown wheels for local mobility. The laser support arm 504 comprises a laser light source 508, a scanner mechanism 510 configured to sweep or scan a laser light beam via an aperture 506 over an area 512. The laser light source is in different embodiments for example an infrared laser in the wavelength range of about 890-1000 nanometers, a CO₂-laser a Nd-YAG-laser, a Dye-laser, GaALAs-laser, Cu-laser or any combination thereof. The scanner mechanism 510 is for example based on a controllably oscillating mirror. In the figure there also illustrating examples of different scanning patterns 512,514,516. The pattern 512 illustrates a laser radiation pattern with a vertical scanning frequency of 50 Hz and horizontal scanning frequency of 0,1 Hz and pattern 514 illustrates vertical scanning frequency of 0,1 Hz and horizontal 50 Hz achieved by means of a square wave scan control signal. Similarly, pattern 516 illustrates a laser radiation pattern with intermittent pauses in the emission of laser light with a vertical scanning frequency of 1 Hz and horizontal 5 Hz.

The operation modes described above are exemplifying embodiments of the invention that are pre-set or pre-programmed in accordance with predetermined control rules. Embodiments of the invention are also possible to adjust during operation for example with regard to emission schemes.

## Claims

1. An apparatus for therapeutic treatment of a living being, comprising
a laser light source (102) configured to radiate a laser light beam on a surface of said living being;
a control unit (104) coupled to the laser light source and configured to control the output of laser light from said laser light source;
a temperature sensor (106) configured to detect the temperature of the radiated surface and coupled to the control unit;
wherein the control unit is configured to control the characteristic of the radiation of laser light on the surface dependent on the detected temperature of the radiated surface **characterised in that** the apparatus further comprises a scanner mechanism configured to scan said laser light beam across a selectable area of said surface.

2. The apparatus of claim 1, wherein a first temperature value of the surface is detected before said laser beam hits the surface, a second temperature value is detected during radiation of said laser beam on the surface and a third temperature value is detected after the laser radiation of the surface has ceased.

3. The apparatus of claim 1, wherein a current temperature of the surface is compared to a predetermined threshold for a maximum allowed temperature.

4. The apparatus of the preceding claim, wherein the radiation intensity is reduced if the maximum allowed temperature is attained.

5. The apparatus of claim 1, wherein the laser radiation is operable such that the temperature is swept in the range of about 35 °C to about 50 °C in an examination phase for the purpose of determining current pain thresholds of a patient.

6. The apparatus of claim 1, wherein the laser radiation is operable such that the temperature is swept in the range of about 35 °C to about 45 °C in a therapy phase for the purpose of stimulating receptors in the skin of a patient.

7. The apparatus of claim 1, further comprising a comparator configured to compare the detected temperature with a predetermined threshold.

8. The apparatus of claim 1, wherein the output of the laser light source is controlled by means of a feedback control loop dependent on the detected temperature.

9. The apparatus of claim 1, wherein the output of the laser light source is controlled such that the detected temperature is kept within a selected temperature range.

10. The apparatus of claim 1, wherein the laser light is pulsed at a pulse rate in the range of about 0,5 to 400 Hertz.

11. The apparatus of claim 1, wherein the laser light is pulsed at a pulse rate in the range of about 80 to 200 Hertz.

12. The apparatus of claim 1, wherein the laser light is pulsed at a pulse rate in the range of about 200 Hertz.

13. The apparatus of claim 1, wherein the laser light is pulsed at a pulse rate in the range of about 1000 Hertz to 10 000 Hertz and the laser light is intermittently interrupted for period in the range of about 1 to 5 milliseconds.

14. The apparatus of claim 1, wherein the laser light is radiated over the surface at a frequency in the range of about 1 to 2 Hertz and controlled to maintain a temperature of about 45°C.

15. The apparatus of claim 1, wherein the laser light is radiated over the surface at a frequency in the range of about 1 to 2 Hertz and controlled to maintain a temperature in the range of about 45 °C to 50°C, with a maximum allowable temperature of about 50°C.

16. The apparatus of claim 1, being operable to radiate the surface with a laser light intensity in the range of about 1 to 5 Watt at a rate of about 200 Hertz and to maintain the temperature in the range of about 40-42 °C.

17. The apparatus of claim 1, being operable to radiate the surface with a laser light intensity in the range of about 5 to 10 Watt at a rate of about 5 Hertz and to maintain the temperature in the range of about 42-43 °C.

18. The apparatus of claim 1, being operable to radiate the surface with a laser light intensity in the range of about 10 to 15 Watt at a rate of about 200 Hertz and to maintain the temperature in the range of about 45°C.

19. The apparatus of claim 1, wherein the laser light is swept over the surface with a predetermined frequency.

20. The apparatus of claim 1, wherein the laser light is swept over a surface area such that the laser light is oscillated around a centre point with an amplitude of about 10-20 centimetres in a first direction and in a second direction, respectively.

21. The apparatus of claim 1, being operated such that the attained surface temperature is swept between a first and a second predetermined temperature, with a predetermined temperature ascend rate and a predetermined temperature descend rate.

22. The apparatus of claim 1, wherein the laser light source comprises one or any combination of a CO₂-laser, a Nd-YAG-laser and a Dye-laser.

23. The apparatus of claim 1, wherein the wavelength of the laser light is 10600 nanometers.

24. The apparatus of claim 1, wherein the wavelength of the laser light is more than 340 nanometers.

## Patentansprüche

1. Vorrichtung zur therapeutischen Behandlung eines Lebewesens, umfassend
eine Laserlicht-Quelle (102), die konfiguriert ist, um einen Laserlichtstrahl auf eine Oberfläche des Lebewesens zu strahlen;
eine Steuereinheit (104), die mit der Laserlicht-Quelle gekoppelt ist und konfiguriert ist, um die Ausgabe von Laserlicht von der Laserlicht-Quelle zu steuern;
einen Temperatursensor (106), der konfiguriert ist, um die Temperatur der bestrahlten Oberfläche zu erfassen und mit der Steuereinheit gekoppelt ist;
wobei die Steuereinheit konfiguriert ist, um die Charakteristik der Bestrahlung des Laserlichts auf die Oberfläche in Abhängigkeit von der erfassten Temperatur der bestrahlten Oberfläche zu steuern, **dadurch gekennzeichnet, dass** die Vorrichtung ferner einen Scanner-Mechanismus umfasst, der konfiguriert ist, um den Laserlichtstrahl über einen ausgewählten Bereich der Oberfläche zu scannen.

2. Vorrichtung nach Anspruch 1, wobei ein erster Temperaturwert der Oberfläche erfasst wird, bevor der Laserstrahl die Oberfläche trifft, ein zweiter Temperaturwert ,während der Bestrahlung des Laserstrahls auf die Oberfläche, erfasst wird und ein dritter Temperaturwert, nachdem die Bestrahlung der Oberfläche aufgehört hat, erfasst wird.

3. Verfahren nach Anspruch 1, wobei eine derzeitige Temperatur der Oberfläche mit einem vorbestimmten Schwellwert für eine maximal erlaubte Temperatur verglichen wird.

4. Verfahren nach einem der vorigen Ansprüche, wobei die Bestrahlungsintensität verringert wird, wenn die maximal erlaubte Temperatur erreicht ist.

5. Verfahren nach Anspruch 1, wobei die Laserbestrahlung betreibbar ist, so dass die Temperatur sich in einem Intervall von etwa 35°C bis etwa 50°C in einer Untersuchungsphase für den Zweck bewegt, die derzeitigen Schmerzschwellwerte eines Patienten zu ermitteln.

6. Vorrichtung nach Anspruch 1, wobei die Laserbestrahlung betreibbar ist, so dass die Temperatur in einem Intervall von etwa 35°C bis etwas 45°C in einer Therapiephase für den Zweck bewegt, Rezeptoren in der Haut eines Patienten zu stimulieren.

7. Vorrichtung nach Anspruch 1, ferner umfassend einen Vergleicher, der konfiguriert ist, um die erfasste Temperatur mit einem vorbestimmten Schwellwert zu vergleichen.

8. Vorrichtung nach Anspruch 1, wobei die Ausgabe der Laserlicht-Quelle durch Mittel eines Rückkopplungs-Regelkreises abhängig von der erfassten Temperatur gesteuert wird.

9. Vorrichtung nach Anspruch 1, wobei die Ausgabe der Laserlicht-Quelle so gesteuert wird, dass die Temperatur in einem gewählten Temperaturintervall gehalten wird.

10. Vorrichtung nach Anspruch 1, wobei das Laserlicht mit einer Pulsrate in dem Intervall von etwa 0,5 bis 400 Hertz gepulst ist.

11. Vorrichtung nach Anspruch 1, wobei das Laserlicht mit einer Pulsrate in dem Intervall von etwa 80 bis 200 Hertz gepulst ist.

12. Vorrichtung nach Anspruch 1, wobei das Laserlicht mit einer Pulsrate in dem Intervall von etwa 200 Hertz gepulst ist.

13. Vorrichtung nach Anspruch 1, wobei das Laserlicht mit einer Pulsrate in dem Intervall von etwa 1000 Hertz bis 10000 Hertz gepulst ist und das Laserlicht periodisch für eine Dauer von etwa 1 bis 5 Millisekunden unterbrochen ist.

14. Vorrichtung nach Anspruch 1, wobei das Laserlicht über der Oberfläche mit einer Frequenz in dem Intervall von etwa 1 bis 2 Hertz abgestrahlt wird und gesteuert ist, um eine Temperatur von etwa 45°C zu halten.

15. Vorrichtung nach Anspruch 1, wobei das Laserlicht über der Oberfläche mit einer Frequenz in dem Intervall von etwa 1 bis 2 Hertz abgestrahlt wird und gesteuert ist, um eine Temperatur im Intervall von etwa 45°C bis 50°C, mit einer maximalen erlaubten Temperatur von etwa 50°C, zu halten.

16. Vorrichtung nach Anspruch 1, betreibbar, um die Oberfläche mit einem Laserlichtintensität im Intervall von etwa 1 bis 5 Watt mit einer Rate von etwa 200 Hertz zu bestrahlen und, um die Temperatur in dem Intervall von etwa 40°C-42°C zu halten.

17. Vorrichtung nach Anspruch 1, betreibbar, um die Oberfläche mit einem Laserlichtintensität im Intervall von etwa 5 bis 10 Watt mit einer Rate von etwa 200 Hertz zu bestrahlen und, um die Temperatur in dem Intervall von etwa 42°C-43°C zu halten.

18. Vorrichtung nach Anspruch 1, betreibbar, um die Oberfläche mit einem Laserlichtintensität im Intervall von etwa 10 bis 15 Watt mit einer Rate von etwa 200 Hertz zu bestrahlen und, um die Temperatur in dem Intervall von etwa 45°C zu halten.

19. Vorrichtung nach Anspruch 1, wobei das Laserlicht über die Oberfläche mit einer vorbestimmten Frequenz bewegt wird.

20. Vorrichtung nach Anspruch 1, wobei das Laserlicht über einen Oberflächenbereich bewegt wird, so dass das Laserlicht um einen Mittelpunkt mit einer Amplitude von etwa 10-20 Zentimetern in einer ersten Richtung beziehungsweise in einer zweiten Richtung oszilliert.

21. Vorrichtung nach Anspruch 1, die derart betreibbar ist, dass die erreichte Oberflächentemperatur zwischen einer ersten und einer zweiten vorbestimmten Temperatur mit einer vorbestimmten Temperaturanstiegsrate und einer vorbestimmten Temperaturabfallrate bewegt.

22. Vorrichtung nach Anspruch 1, wobei die Laserlicht-Quelle einen oder eine beliebige Kombination von einem CO₂-Laser, einem Nd-YAG-Laser und einem Farbstoff-Laser umfasst.

23. Vorrichtung nach Anspruch 1, wobei die Wellenlänge des Laserlichts 10600 Nanometer beträgt.

24. Vorrichtung nach Anspruch 1, wobei die Wellenlänge des Laserlichts mehr als 340 Nanometer beträgt.

## Revendications

1. Appareil pour le traitement thérapeutique d'un être vivant, comprenant :
une source de lumière laser (102) configurée pour rayonner un faisceau de lumière laser sur une surface dudit être vivant ;
une unité de commande (104) couplée à la source de lumière laser et configurée pour commander la sortie de la lumière laser de ladite source de lumière laser ;
un capteur de température (106) configuré pour détecter la température de la surface rayonnée et couplé à l'unité de commande ;
dans lequel l'unité de commande est configurée pour commander les caractéristiques du rayonnement de lumière laser sur la surface en fonction de la température détectée de la surface rayonnée, **caractérisé en ce que** l'appareil comprend en outre un mécanisme de balayage configuré pour balayer ledit faisceau de lumière laser sur une zone pouvant être sélectionnée de ladite surface.

2. Appareil selon la revendication 1, dans lequel une première valeur de température de la surface est détectée avant que ledit faisceau laser ne heurte la surface, une deuxième valeur de température est détectée pendant le rayonnement dudit faisceau laser sur la surface et une troisième valeur de température est détectée après que le rayonnement de laser sur la surface a cessé.

3. Appareil selon la revendication 1, dans lequel une température courante de la surface est comparée à un seuil prédéterminé pour une valeur maximum admissible.

4. Appareil selon la revendication précédente, dans lequel l'intensité de rayonnement est réduite si la température maximum admissible est atteinte.

5. Appareil selon la revendication 1, dans lequel le rayonnement laser peut fonctionner de sorte que la température est balayée dans une plage de l'ordre d'environ 35 °C à environ 50 °C dans une phase d'examen afin de déterminer les seuils de douleur courants d'un patient.

6. Appareil selon la revendication 1, dans lequel le rayonnement laser peut fonctionner de sorte que la température est balayée dans la plage de l'ordre d'environ 35 °C à environ 45 °C dans une phase thérapeutique afin de stimuler des récepteurs dans la peau d'un patient.

7. Appareil selon à revendication 1, comprenant en outre un comparateur configuré pour comparer la température détectée avec un seuil prédéterminé.

8. Appareil selon la revendication 1, dans lequel la sortie de la source de lumière laser est commandée au moyen d'une boucle de commande de rétroaction en fonction de la température détectée.

9. Appareil selon la revendication 1, dans lequel la sortie de la source de lumière laser est commandée de sorte que la température détectée est maintenue dans une plage de températures sélectionnée.

10. Appareil selon la revendication 1, dans lequel la lumière laser est pulsée à une vitesse d'impulsion de l'ordre d'environ 0,5 à 400 Hertz.

11. Appareil selon la revendication 1, dans lequel la lumière laser est pulsée à une vitesse d'impulsion de l'ordre d'environ 80 à 200 Hertz.

12. Appareil selon la revendication 1, dans lequel la lumière laser est pulsée à une vitesse d'impulsion de l'ordre d'environ 200 Hertz.

13. Appareil selon la revendication 1, dans lequel la lumière laser est pulsée à une vitesse d'impulsion de l'ordre d'environ 1 000 Hertz à 10 000 Hertz et la lumière laser est interrompue de manière intermittente pendant une période de l'ordre d'environ 1 à 5 millisecondes.

14. Appareil selon la revendication 1, dans lequel la lumière laser est rayonnée sur la surface à une fréquence de l'ordre d'environ 1 à 2 Hertz et commandée pour maintenir une température d'environ 45 °C.

15. Appareil selon la revendication 1, dans lequel la lumière laser est rayonnée sur la surface à une fréquence de l'ordre d'environ 1 à 2 Hertz et commandée pour maintenir une température de l'ordre d'environ 45 °C à 50°C, avec une température maximum admissible d'environ 50 °C.

16. Appareil selon la revendication 1, pouvant être actionné pour rayonner la surface avec une intensité de lumière laser dans la plage de l'ordre d'environ 1 à 5 Watt à une fréquence d'environ 200 Hertz et pour maintenir la température dans la plage de l'ordre d'environ 40 - 42 °C.

17. Appareil selon la revendication 1, pouvant être actionné pour rayonner la surface avec une intensité de lumière laser de l'ordre d'environ 5 à 10 Watt à une fréquence d'environ 5 Hertz et pour maintenir la température dans la plage de l'ordre d'environ 42 - 43 °C.

18. Appareil selon la revendication 1, pouvant être actionné pour rayonner la surface avec une intensité de lumière laser de l'ordre d'environ 10 à 15 Watt à une fréquence d'environ 200 Hertz et pour maintenir la température dans la plage de l'ordre d'environ 45 °C.

19. Appareil selon la revendication 1, dans lequel la lumière laser est balayée sur la surface avec une fréquence prédéterminée.

20. Appareil selon la revendication 1, dans lequel la lumière laser est balayée sur une surface de sorte que la lumière laser est oscillée autour d'un point central avec une amplitude d'environ 10 - 20 centimètres dans une première direction et dans une seconde direction, respectivement.

21. Appareil selon la revendication 1, pouvant être actionné de sorte que la température de surface atteinte est balayée entre une première et une seconde température prédéterminée, avec un taux ascendant de température prédéterminé et un taux descendant de température prédéterminé.

22. Appareil selon la revendication 1, dans lequel la source de lumière laser comprend l'un ou n'importe quelle combinaison parmi un laser à CO₂, un laser à Nd-YAG et un laser à colorant.

23. Appareil selon la revendication 1, dans lequel la longueur d'onde de la lumière laser est de 10 600 nanomètres.

24. Appareil selon la revendication 1, dans lequel la longueur d'onde de la lumière laser est supérieure à 340 nanomètres.
